# EUROPEAN PATENT APPLICATION

(11) **EP 1 006 362 A1**
(43) Date of publication of application: **07.06.2000**
(21) Application number: 98122848.9
(22) Date of filing: 02.12.1998
(51) Int. Cl.: G01N 33/53, G01N 27/447

(54) **Method and apparatus for the separation of components from a biological material**

(71) Applicant: Cahill, Michael, Dr., 72116 Moessingen (DE); Drukier, Andrzej, Dr., Burke, VA 22015 (US); Nordheim, Alfred, Prof. Dr., 72135 Dettenhausen (DE)
(72) Inventor: Cahill, Michael, Dr., 72116 Moessingen (DE); Drukier, Andrzej, Dr., Burke, VA 22015 (US); Nordheim, Alfred, Prof. Dr., 72135 Dettenhausen (DE)
(74) Representative: Patentanwälte Ruff, Beier, Schöndorf und Mütschele

(57) **Abstract**

This inventive method provides an essential improvement for the analysis of complex mixtures of heterogeneous components. The principle is based on a prefractionation of the mixture to be analyzed in accordance to the relative abundances of the individual components of the mixture. The following analysis of the mixture is performed in separated fractions, which contain components of same or similar abundance. In a preferred embodiment of the invention the components are proteins or peptides, which are analyzed in the field of proteomics. However an employment of the inventive method for a large variety of molecules including lipids, hydrocarbon-based molecules, and nucleic acids will be possible for those skilled in the art.

## Description

The invention relates to a method for separation of components from a material to be analysed, in particular of complex mixtures of biological molecules.

In a plurality of biochemical and/or biomedical applications, including application for genetically modified foodstuffs, the mixtures of a very large number of analyte molecules, say more than 5,000 different species, are present. A good example can be provided by a collection of all of the proteins from a given cellular line, a given tissue, or a given organism. It is important to understand that in such collections one observes not only a very large number of clearly definable species, but also large differences in abundance between these said species. Alas, contrary to intuition, in many biomedical situations the economical importance of biological function does not necessarily correlate with abundance. Actually, it is currently believed that a very large proportion of switch molecules which regulate the networks of protein activity within cells have a very low abundance. A good example is given by the proteins of yeast which have been studied extensively. Out of about 6,000 proteins expected from the known yeast genome, only about 2,000 proteins have been reliably detected/quantified. However among the proteins which have been identified, there are relatively few examples of transcription factors, a crucial class of proteins which regulate gene activity.

Another poignant example can be drawn from the field of oncology. Cancerous cells have escaped the rules which govern ordered and controlled cell proliferation within the organism. Among these controls are the telomeres, which are cap-like structures to be found at the extreme termini of chromosomes. The number of telomeres possessed by a chromosome within a cell determines the number of times the cell may divide, as one telomere is lost per cell division. Thus, to escape normal growth controls, cancerous cells must escape from the telomere copy number control. This is possible if the cell contains an enzymatic activity, telomerase, which can add new telomeres to a chromosome. Conceivably, the aberrant presence of a single or a few telomerase enzyme complexes per cell could carry a pre-cancer cell through a crisis period, enabling continued cell division until a compensating mutation alleviated telomere dependance, creating a vigorous and clinically important cancerous cell.

The above examples underline the importance of proteomics, which is the study of all proteins expressed by a genome of the cells under study. The acronym "PROTEOME" (PROTEins within a genOME) [Wilkins, M. R., et al. (1996). From proteins to proteomes: large scale protein identification by two-dimensional electrophoresis and amino acid analysis. Bio/Technology 14, 61-65] was coined by Marc Wilkins from Sydney at the 1st Siena 2D Electrophoresis Meeting in 1994. Proteomics may eventually reveal the identity and regulation of most proteins expressed within a particular cell-type [Wilkins, M. R., et al. eds (1997). Proteome Research: New Frontiers in Functional Genomics (Berlin: Springer) 243 pp]. However the sensitivity and resolution currently attainable require improvement before this can be accomplished.

Because proteomics requires detection and quantitation of a very large number of different species, it is necessary to use the available separation methods. The preferred tools of proteomics are currently 2D electrophoretic gels for detection and quantitation, followed by a plurality of methods for protein identification, including amino acid analysis, Edman degradation, isoelectric point, apparent molecular mass, and mass spectrometry. There exist a plurality of conceivable two dimensional separation techniques, but currently the most performant is the one in which the first separation dimension utilizes differences in isoelectric point, and the second differences in molecular mass. An intrinsic strength of 2D gels is that they can reveal molecules which undergo post translational modifications, such as phosphorylation, geranylation, glycosylation, proteolytic cleavage, translational induction, alternative splicing, or any modification that affects molecular weight or isoelectric point. Current proteome technology essentially corresponds to a differential display on the protein level and complements the nucleic acid-based method. An overview of the recent state of the field has been recently published [Wilkins, M. R., et al. eds (1997). Proteome Research: New Frontiers in Functional Genomics (Berlin: Springer) 243 pp].

In the following there is a list of main challenges which are currently impeding proteomic studies.
A: Protein solubility challenges.
B: The complexity issue.
C: 2D Gel connected issues.
D: The protein identification issue.
E. The Bioinformatics issue.

Proteome studies require denaturing conditions for the first isoelectric focussing dimension. Denatured proteins behave as monomeric entities, thus reducing complexity of the readout which would arise if one molecule were involved in a variety of heteromeric intracellular protein complexes, each with a unique isoelectric point. However the buffer components which induce protein denaturation must themselves be neutrally charged or else their function would vacate the isoelectric focussing dimension. Thus these molecules must be either uncharged, or of a zwitterionic nature with pK of the ionizing groups at least two pH units outside of the pH range to be examined by isoelectric focussing. These limitations have restricted the reagents (surfactants and chaotropic agents) used for classical isoelectric focussing to urea, or more recently a mixture of thio urea and urea, as chaotropic agent, and to nonionic detergents or sulfobetaine-like zwitterions such as CHAPS (3-[(3-cholamidopropyl)dimethylammonio]-1-propane-sulfate). It has been suspected that these molecules provide non-optimal solutions to the problem of solubilizing as many proteins as possible. Indeed none of the 15% theoretically most hydrophobic protein open reading frames predicted from the genomic sequence of the bacterium Escherichia coli were observed in 2D data bases [Wilkins, M. R., et al. eds (1997). Proteome Research: New Frontiers in Functional Genomics (Berlin: Springer) 243 pp].

Additionally, solubilization of the insoluble pellet from 8M urea, 4% CHAPS buffer into urea/thiourea buffers containing nonconventional sulfobetaine-like molecules resulted in the appearance of previously undetected proteins in 2D gels. Thus the fraction of total gene products analyzed by proteomic studies is currently far from optimal. With increased efficiency of protein solubilization will come an accompanying increase in the complexity. This in turn requires improved methods for systematic and quantitative analysis.

One central problem is that the two dimensional gels currently used for proteomic research typically visualize between 2,000 and 10,000 proteins per gel, depending on laboratory and sample type. It is estimated that the human genome encodes between 50,000 to 100,000 functional genes. Of these, between 5,000 - 15,000 are thought to be expressed in single cell-types, and with multiply spliced mRNAs, the number of different spots to be expected on a two dimensional gel may be around 50,000. While these estimates probably contain large errors, current technologies may visualize in the vicinity of 5-20% of existing proteins, and the majority of these may be highly expressed structural and "housekeeping" proteins. This means that perhaps greater than 80% of the proteins of interest are currently undocumented by proteome analysis. As the human proteome can only be partially estimated from genome sequence, it may be appropriate to remember that the functional protein complement is largely evolutionarily conserved. Thus, it is possible to argue from model organisms, such as yeast or fruit fly, about the dynamic range which might be expected in human situation. The entire genome sequence of the yeast Saccharomyces cerevisiae has been determined. About one half of predicted proteins have been identified, and their quantity measured. The yeast data suggest that the first 100 most frequent proteins account for 50% of total protein, and a dynamic range of 100,000 is observed between the most frequent (high abundant or in the following abundant) and the least frequent (low abundant) protein detected. Detection of low abundance proteins is currently limited by instrumental background, so there is no estimate of the actual true dynamic range possible.

In order to present some scale to the problem involved in visualizing as many proteins as possible in proteome analysis of human cells, consider a typical cultured B-Cell protein extract. The human genome contains many more open reading frames, which encode for proteins, than yeast. Thus the dynamic range is likely to be greater. Approximately ten million cells are required to visualize one or two thousand protein spots when stained with Coomassie Blue, or one million cells with silver staining, which is the prior-art most sensitive method available which does not require metabolic labelling of the cells. Western blot studies have demonstrated that there are lower abundance proteins present in such gels which are not visible with these techniques. Now, assume that an abundant 50 kDa protein comprises for simplicity sake 1% of all proteins. If one million cells contain 100 µg protein, and thus 1 µg of this 50 kDa protein, then this molecule is present at 20 picomole/ million cells (1 picomole = 10⁻¹² moles), or 20 attomole/cell (1 attomole = 10⁻¹⁸ mole). There are 6 x 10²³ molecules per mol, so a single cell has 12 million of these 50 kDa proteins, which appears as a prominent silver-stained spot.

This invention facilitates the task to provide solutions for an improvement of analysis of complex mixtures of molecules. The problem is solved by a method and apparatus comprising the features of claim 1 or claim 25 to 28, respectively. Preferred embodiments of the inventive method are shown in claims 2 to 24. The wording of all claims is hereby made to the content of the specification by reference. The different embodiments of the invention mainly concern macromolecules such as proteins, but the invention is not limited to that kind of biological molecules.

Considerable progress, especially in proteomics, can be obtained by providing a specific or a new combination of specific steps according to the inventive method. Thus, the key enabling capability of this invention is a removal of abundant proteins, resultant alleviation of local gel overheating and associated diffusional loss of resolution, and/or by using the most sensitive imaging and quantitation methods possible, e.g. use of the MPD detectors.

By reference to "abundance" the relative frequency (amount) of a component in a mixture is meant, wherein in the following "abundant molecules" is meant as molecules, which are comparatively or very frequent and "low abundant molecules" is meant as molecules, which are comparatively or very rare in the mixture. The boundary between "abundance" i.e. "high abundance" and "low abundance" has to be defined according to the circumstances, e.g. the most frequent molecules of a mixture, which account for e.g. 50% of total molecule mass, could be defined as high abundant, i.e. abundant. Furthermore, the boundary between abundance and low abundance could be defined as an absolute amount of a component, e.g. 500 fmole.

By use of the prior art Multiple Photon Detector technology (MPD: e.g. US Patent Number 5532122) a detection limit of much better than one attomole has been achieved for proteins. MPD is a technique for the detection and measurement of certain radioisotopes at activity levels which are much below the naturally occurring background. This is made possible by the nearly perfect rejection of background events which is achieved through sophisticated signal processing and analysis. Certain isotopes emit both X-rays as electrons move to lower orbitals, and gamma rays as neutrons decompose upon radioactive decay. The high degree of sensitivity of MPD is obtained by considering only signals with coincident X- and gamma-rays, and where the energy profile of the measured photons correspond to those expected for the isotope in question. The isotopes compatible with MPD come from two broad families of radioisotopes which together include over 100 members appropriate for use as tags. In particular, they include the isotope iodine-125 (¹²⁵I), one of the most commonly used isotopes in biomedical diagnostics. By carefully comparing each detected event with the highly specific decay signature of the used radioisotope, MPD is able to reduce the number of background events to less than one per week. MPD permits multi-labeling (up to 16 co-resident labels), good spatial resolution (about 0.3 mm) and very high dynamic range (linearity over nine orders of magnitude in label down to 10⁻²¹ mole). Two significant advantages which are derived from this capability are that substances labeled with appropriate isotopes can be reliably detected at four orders of magnitude lower concentrations, and the amount of isotope which must be used is reduced 1,000-fold to a level much below the naturally occurring background radiation.

Using MPD, a signal arising from 600,000 molecules or more could be visualized. Therefore by examining one hundred thousand cells per experiment it would be possible to visualize molecules present in the cell as a single copy per cell. However before this is possible an assay and visualization system must be achieved with a dynamic range of 10 million or more, and a resolution of 50,000 separate data points. This represents a formidable technical and logistical task for future developments. The problems responsible for this poor performance may be reduced to several areas, including: detection sensitivity, spatial resolution and ability to handle very high dynamic ranges, including the problems of global and local overload.

This patent discloses techniques permitting separation of highly abundant proteins, which may occupy significant portions of the surface area of broad range pH isoelectric focussing gels, and thus would obscure from detection minor components which comigrate on two dimensional gels. Until the present this has been technologically impossible. Ideally such a system will offer thousands of separation windows, so that as many as possible of the separated analytes would migrate in their own window. Of the available technologies, in the context of the invention capillary electrophoresis (CE) has a very promising separation potential on a micro scale, particularly when coupled with some prior-art methods of resolving sample complexity. Having detected an analyte peak, the fractionation device will be able to divert peaks to one or many storage receptacles, when peak intensity is detected over a predetermined threshold value. The load volumes for CE are in the order of 0.1-0.4 microlitres (µl), such that a detector to discriminate between peaks has to be extremely sensitive. The present possibilities, without modifying the proteins, is UV detection at 260/280 nm for tryptophan and tyrosine, or 215 nm for the peptide bond, but for very low protein concentrations neither of the UV methods is sensitive enough. Also, ³⁵S labelling of cells is not sensitive enough for such low protein concentrations due to the short passage time through the detector. Additionally, present detection of proteins requires the loading of protein amounts large enough to extend peak breadth, reducing the number of resolution windows available. Thus, the lower loading amounts permitted by the preferred MPD detection will increase the number of windows of resolution analogous to peak narrowing in either HPLC or CE when less material is loaded.

It is mentioned that the performance of 2D gels is dependent on a plurality of preparative steps. Innovative preparative steps as disclosed in this patent are applicable to sample preparation for two dimensional gels, but are by no means restricted to such applications. It is clear that these concepts will provide useful tools for analyte separations in combination with a variety of other separation pinciples, the particular combination being chosen to be optimal for the sample in case, and the question to be analyzed.

This patent discloses a method which will permit at least partial solutions to the complexity issue and thus permits simplifications and improvements in subsequent separation and protein detection, such as that currently performed using 2D gel. However this method is independent of 2D gels and could be applicable to other technological developments. Note that a subset of the concepts disclosed here are not meant to replace two dimensional gel technology at this stage, however such a development is possible. Currently, the disclosed method will be used for the reproducible detection of analytes, and their reliable dispensation to an appropriate fraction collection space.

There are essentially two classes of situation which would be encountered in proteomic studies.
- Class 1:: the available protein is practically unlimited (e.g. cells are cultivatable in vitro).
- Class 2:: the amount of available proteins is very small, e.g. when one is interested in forensic material or for low volume clinical material.

The ultimate case of the second class are studies of protein contents of single cells or organelles.

In the class 1, the notion of a global overloading of the system of separation, e.g. a 2D gel is important. Typically current detectors can reliably detect and quantitate a spot at about a femtomole level. Taking into account the dynamic ranges of protein abundance may be as large as million, this would lead to a loading requirement of up to a few hundred nmol of proteins to a gel to allow detection of low abundance proteins. This would lead to a total overload of a broad pH range IPG (immobilized pH gradient) isoelectric focussing gel, and often to very serious streaking artifacts in both dimensions. Loading the sample to narrow range IPGs would alleviate these problems for large domains of the gel, however the high abundance components would still occupy their preferred pH values, introducing the above mentioned local artifacts to these gel regions.

One could think of overcoming the global overloading issue by an improvement of sensitivity of the imaging detectors. For example, MPD technology permits imaging at sub-attomole per spot level, which may lead to a few hundred picomol (or less) per gel total load requirements for unfractionated cell proteins, which considerably removes global overloading. This principle applies not only to electrophoretic, but also to chromatographic and other separation methods.

However even the use of the most sensitive detectors alone, like MPD imagers, does not solve problems due to local overloading. This is shown by the following discussion of spot size, local overloading, and local overheating. Just as increasing the ionic strength of the gel buffer increases current in an electric field, increasing the ionic strength by introducing concentrated macromolecules such as nucleic acids or proteins will increase local temperature. Reasonably this effect may lead to larger spots in globally or locally overloaded protein 2D gels. The effect will be a function of diffusion induced by increased temperature. One could circumvent this by loading less analyte molecules. However this would decrease the concentration of the protein in the area over which the spot is distributed.

The local temperature of a gel may limit both diffusion and mobility of a given protein in a gel. Thus any local overloading which may happen at nanomol per mm² level is a latent risk for reliable high spatial resolution 2D gels. A partial solution to this problem is obtainable by optimization of the gel running condition. One takes into consideration both the gel overheating characteristics, and the fact that a diffusion limited spot size is proportional to the size of the time of running the gel. The optimal condition, the condition at which spot size is minimized, may be different for high abundance and low abundance spots. Thus it may be possible to obtain gel running conditions which would improve the spatial resolution of the gel for low abundance spots. Even a small improvement in resolution, e.g. factor two, leads to a quite large improvement in number of spaces resolved, i.e. factor four. Because there exists a serious limitation on the sensitivity of imaging detectors, the current strategy in proteomics is to run all proteins together and detect all proteins down to the limit of the sensitivity of the detector. MPD enables visualization down to sub attomole level, and a dynamic range of up to 9 orders of magnitude. The gel however, sustains only 4-5 orders of magnitude dynamic range because of local overheating effects. Therefore there is a certain advantage to remove most abundant proteins according to the invention. The new strategy proposed comprises analyzing separately subsets of proteins, each selected according to their relative abundance. Each of these subsets will be run separately, with optimized gel running conditions. This allows maintenance within each subset of a number of analytes which are within the resolution capacity of the chosen separation system. For example for 2D gels, within each subset the dynamic range would be kept to 2 or 3 logs so as to diminish artifacts due to overload and overheating.

A conventional current wisdom among proteome practitioners is that to see low abundance proteins, many gels of narrow pH range have to be performed. This solution is currently non optimal since it addresses neither the linear dynamics nor local overloading problems. For instance any protein which co-purifies with an abundant protein such as albumin will never be detected. This patent discloses that a preparatory method, i.e. a prefractionation by abundance, is used, especially in view of the enabling super-sensitivity of imaging detectors like MPD. The disclosed method can be automated, minimizing the effort expended by the experimenter.

The use of a method as described here in studies of heterogenous mixtures of biological molecules, such as in proteomics, will enable visualization of low abundance analytes, such as proteins, by separating them from higher abundance molecules which may otherwise shield their presence. The effect of application of this technology on proteomic studies will be analogous to that of the Hubble telescope on astronomy, looking past the brighter signals to reveal details of the many fainter signals.

The technical means exist to perform the sample evaluation without introducing contamination and non-specific biological losses during detection of very low abundance proteins. The precedures proposed here provide initial progress towards identifying very low abundance cellular proteins. There exists a considerable market demand for such procedures. The invention is the approach to the solution of the problems of numerical complexity and linear dynamics by providing a step of prefractionation by abundance, preferably with subsequent separate analysis of abundance classes with suitable levels of sensitivity. Some of the possible methods for pre-gel selection of protein abundance are disclosed. The scope of this patent is, however, not only in the particular implementation of selection referred to in this description, but in the recognition of the fact that the preparative steps of removal of abundant proteins permits a new systematics of innovative strategies of separation of the analytes into resolution windows of the separation system into a grid-like format, such as that presented by separation on different 2D gels for each abundance class.

### Affinity based application

In a first preferred embodiment of the invention the removal of abundant biological molecules is performed using immunological means, i.e. affinity reagents. Another preferred possibility is a fractionation in appropriate separation media, but one advantage of the immunological method is the potential for very high specificity. In a further preferred embodiment the immunological method comprises the following steps:
1: (Optional) An initial low sensitivity 2D gel to identify the most abundant proteins in the sample.
2: Provision/Creation of antibodies or other macromolecules or a collection of antibodies or other macromolecules which specifically bind to the above mentioned abundant proteins, and importantly, which preferentially bind to only one protein per spot.
3: Affinity purification of the protein sample.
4: Subsequent high sensitivity 2D gel optimized for high spatial resolution for low abundance proteins after removal of selected abundant molecules.
5: High sensitivity imaging of the above said 2D-gel, e.g. using MPD imagers.

Obviously, in generalization of the above said procedure, any other high resolution protein separation method can be used instead of 2D gel electrophoresis. In a preferred variant of this process, the molecules with affinity to abundant proteins are the prior art aptamer short nucleic acid molecules. These can be amplified and selected with high efficiency at low cost.

In a preferred embodiment of the invention a low sensitivity 2D gel is used to identify the proteins in the sample. The abundant spots are then removed and are used in the subsequent steps of antibody/aptamer selection. Each of the proteins could be identified by Mass Spectrometry or other methods. This information could be used for the selection of the appropriate antibody/aptamer from a catalogue or the proteins themselves could be used to generate antibodies, e.g. in the mouse or in the rabbit.

In one implementation a very large library of antibodies or aptamers exists, e.g. a few thousand antibodies or molecules with affinity to the proteins of interest, and this library is challenged with the protein mixture of interest. The library is organized randomly or preferred into an array of spatially distinguishable pixels, e.g. on a chip. Fluid containing the protein analytes is incubated with or passed over said chip. This is in result a type of micro affinity chromatography, where according to the invention low abundant molecules of interest remain in the fluid. Abundant targeted molecules could be recovered from the affinity array. In one embodiment such an affinity chip is challenged with the collection of abundant proteins cut from the gel and marked isotopically. The protein interactions with specific antibodies create a spatial pattern which is read-out with an appropriate detector. Herein the use of the MPD technique is preferred, which permits sensitive detection with antibodies which do interact strongly with the protein mix of interest. Additionally, the number of proteins which were not trapped (e.g. in the supernatant) can be estimated from the mix (e.g. from the supernatant).

In one embodiment of this inventive aspect the said antibodies or the said collection of antibodies consist of monoclonal antibodies. In this case the probability that two proteins will bind to the same antibody is very small. Similarly, the probability that a given protein will bind to two antibodies is also small, and is a function of the number of antibodies employed. For example, and preferably, using MPD imagers this binding process is quantitative even at an attomole level. It can be used for selecting the antibodies to proteins present in the gel at sub-femtomole level. Herein, e.g. it is assumed that only about 10% of total amount of protein is loaded in the "trial 2-D gel". By comparing the amount of protein binding to antibody for each pixel with the abundance of each of the spots removed from the 2D gel will provide information about the antibody/protein pairing. This information could be improved if the antibody array is challenged sequentially, with the measurement step after each challenge. It is also possible to use polyclonal antibodies in order to detect and remove high abundant molecules.

A very efficient strategy to create a large collection of affinity reagents according to this invention is using a library of high abundance proteins to evolve a collection of highly specific affinity reagents. Two implementations of this concept are disclosed which involve evolution of a specific set of either aptamers, or polypetides through the phage display. Aptamers are the nucleic acid constructs evolved through the SELEX process as described in a plurality of prior art patents and papers (e.g. US Patent No. 05567588; US Patent No. 05637459; Tuerk, C., et al. (1992) PNAS 89: 6988-6992). For the purposes of this invention it is not distinguished between antibodies grown by classical immunological methods, and highly specific polypeptides grown by the phage display method. It will be seen in the following disclosure that a process of evolution of such a set of specifically interacting molecules involves the step of exponential but selective amplification. Because of the exponential nature of this amplification the selected aptamer/antibody will be towards a dominant protein, and not towards any low abundance proteins co-located with the peak of abundant molecules. To illustrate this possibility, the case of aptamers obtained through the SELEX process is considered. Herein a combinatorial library of 10¹⁵ different nucleic acids is concurrently challenged with a single target protein. Typically only about 10-100 copies of each of the nucleic acids is used. If only 10% of them efficiently binds to the target, the nucleic target is amplified a billion fold. By repeating this process a few times, very specific aptamers can be evolved in a short time. If challenged by a dominant protein in the spot this selection process would lead to only a few candidate nucleic acids, and the probability of binding to a low abundance protein in the same peak would be very small.

Due to the possibility of efficient amplification of nucleic acid structures the production of aptamers specific to a given molecular target is relatively low cost as compared with classical immunological methods. Optionally, a series of libraries of aptamers specific to the dominant proteins for a given model organism, organ, type of tissue, or cellular line, can be efficiently and economically created. These arrays could be used as a general tool for affinity purification and removal for almost all the most abundant proteins in a given application.

Both the techniques of aptamer selection and of phage display are prior art techniques, however the use of these techniques for rejection of highly abundant proteins preselected from one or more 2D gels is innovative. Also claimed in this respect is this use on highly abundant proteins selected by abundance using the CE/MPD device described in this patent. Specifically the creation of collections of aptamers targeting typical dominant proteins from protein sources of interest is disclosed because of its economical advantages. The examples of some such targeted sources of proteins are model organism, such as Nematodes, Drosophila, yeast, Zebra fish, amphibians, mammals, and humans. Concerning specific tissues, reference is made to the subsets of aptamers targeted to abundant proteins from tissues such as lymphocytes, liver, nervous tissue, skin, and bone tissues such as bone marrow or cartilage. With elucidation of proteomes of further cell types and tissues, further entries could be added to the library of aptamer subsets.

The device proposed should separate abundant analytes, such as proteins, from less abundant ones in a complex mixture, such as proteins extracted from primary human clinical biopsies. By differentially examining the abundant and non abundant components with appropriate sensitivity, additional biological information should be harvested which is unobtainable using current technology.

The described features and further features of the invention are described in greater detail below by way of example with reference to variants shown in diagrammatic form.

### 2D gel based application

A preferred embodiment of the subject invention is an innovative procedure for separating proteins by abundance, wherein two subsequent 2D-gels are obtained. The first, preparative, gel is used to obtain the realistic estimation of the number of abundant proteins, i.e. proteins above a certain abundance threshold. In the following it is assumed that this threshold is above 1 picogram of protein per spot (1 pg/spot). However, even lower thresholds, say down to 100 fg/spot (100 femtogram of protein per spot), can be useful in certain applications. The said 2D gel image is then analyzed either manually or with means of computer generated representation. The first preparative 2D gel can be of lower spatial resolution and hence can be run much more quickly than an analytical 2D gel. Also, when the MPD instrumentation is used to analyze the preparative 2D gel, the read-out time is much faster than for a fully resolved second 2D gel. A main innovative step is to localize and physically remove a large fraction, typically 90% of each abundant protein, from the preparative gel. This can be achieved by either mechanically cutting a part of the gel or by using local electroelution to remove the selected proteins. Also, evaporation by laser beam is disclosed. After this abundant protein removal step, all "below the threshold" proteins are removed from the gel, either by dissolving the gel or by electroelution. The collection of such "below the threshold" proteins is then analyzed by means of another 2D gel.

A preferred embodiment of the invention consists of the following steps:
a) preparation of preparative 2D gel;
b) analysis of the said preparative 2D gel permitting identification of all high abundance protein spots;
c) removal of the said high abundance protein spots;
d) recuperation of low abundance proteins from the gel;
e) preparation of high resolution 2D gel using only low abundance proteins;
wherein the step of the high abundance protein spot removal is performed in such a way, that a small fraction of the spot, say a few percent, remains on the gel.

Fortunately, in the process of 2D gel preparation and analysis, there are two easily distinguishable cases of pre-gel and post-gel derivatization. In the case of pre-gel derivatization there are four distinguishable steps:
1) protein preparation;
2) pre-gel derivatization;
3) electrophoretic process;
4) gel imaging.

Typical pre-gel derivatization processes are either radiolabeling or biotinylation of proteins.

In the case of the post-gel derivatization, the steps of the procedure are:
1) protein preparation;
2) electrophoretic process;
3) post-gel derivatization;
4) gel imaging.

The post-gel derivatizations are currently more popular and include staining with Coomassie Blue, silver staining or use of fluorescent dyes. It is noted that the above said stains lead to a large change of mobility of the derivatized proteins. Thus, it could be difficult to use them in the proposed method of abundant proteins elimination. Both Coommassie Blue and silver stain binds to proteins reversibly and could be removed, but this could lead to irreproducible losses of proteins and hence be difficult to practice.

The manual processing of the gel is clearly feasible but can be difficult, if the total number of high abundance spots is not relatively small, say less than one hundred. In this case, the use of manual, razor blade based methods or procedures using one cylindrical cutter or a special set of cylindrical cutters of different diameter is disclosed.

Another disclosed implementation uses an automatic X-Y-Z (coordinates) computer based mover. The automatic arm is moved into position under control of a computer and is placed above the spot identified as a high protein abundance spot. The spot diameter is evaluated, and a cylindrical cutting tool of appropriate diameter is selected. Typically, the cutter diameter is selected after the computer analyzes the density within a spot and calculates the diameter which will remove the preset fraction of the total amount of the proteins in the spot. To facilitate material removal, vacuum can be applied for a short period of time while the cutting tool is in contact with the 2D gel. Typically, the thickness of the gel is sufficiently uniform so that a single Z displacement mechanism can be used. However, the use of special sensors to establish when the cutting tool touches the gel is disclosed. The said sensor can be either optical or electrical, i.e. taking account of the electrical conductivity of the gel or in the case of capacitive sensors, the dielectric constant of gel medium. The problem of contamination can be a challenge in the case of repetitively used mechanical cutter tools. Preferably, after each excision, the tool can be carefully washed and dried. In a proposed automatic system for each spot a cutter tool with different diameter could be selected. In this inventive implementation, the computer analyzes the 2D gel and classifies all high protein abundance spots according to their diameter. Then a cutter tool with, say, the largest diameter is selected and mounted on X-Y-Z arm. This cutter tool is sequentially displaced over the surface of the 2D gel until all high abundance protein spots are excised. Afterwards, the other cutting tool with a smaller diameter is selected. The process continues until the desired set of cutting tools is used to excise all high abundance proteins spots. Obviously, the order of use of cutter tools of different diameter is immaterial. One could e.g. as well start with the cutter tools of smallest diameter.

Another innovation disclosed in this patent, is a use of variable diameter cutter tool connected to the disclosed X-Y-Z mover steered by the computer analyzing the preparative 2D gel. Further, when using the above described X-Y-Z mover, the mechanical excision using a set of the mechanical cutters can be replaced by the electroelution of the proteins from high abundance spots. Herein, an appropriate semi-permeable electrolution probe, typically a wick, is used. When it touches the gel surface and is placed under electric voltage, it will move the proteins from the gel through the electroelution probe towards an appropriate receptacle. The amount of mobilized protein is roughly proportional to elution time, and can be well controlled electronically using feedback from a computer. Herein, information about spot position is required, and permits use of known molecular size, or distance migrated in the 2D gel, to calculate the mobility of the proteins. This information allows calculation of the appropriate electroelution conditions. Such an electroelution probe is favorable because it is self-cleaning, i.e. all proteins are efficiently removed from the electroelution probe by appropriate voltage. Thus, the process of removal of all high abundance spots is very fast because e.g. no cleaning step is required.

Yet another process uses the X-Y(-Z) mover and a high intensity pulsed laser beam. The gel is placed in the vacuum chamber. A laser beam is directed towards the high abundance protein spot. A very short but intense laser beam is used to explosively evaporate the gel. The evaporated gel material takes with itself the proteins. The remaining low abundance proteins are liberated from the gel and the high spatial resolution 2D gel is run and analyzed. In another implementation, instead of an X-Y(-Z) mover, an appropriate light deflector is used. Herein, the laser beam is moved by means of a mechanical moving mirror or by means of an electro-optical deflector, e.g. using Kerr effect, towards any spot on the surface of the 2D gel.

To facilitate such explosive evaporation, the proteins preferably are transferred onto a membrane. For that a nitrocellulose membrane with an admixture of nicotinic acid is preferred and an infrared laser is used.

Another preferred embodiment of the subject invention uses electroelution and a special preferably computer generated impermeable mask. For example, a thin plastic or rubber with appropriate hole patterns can be used. This pattern is generated by laser beam or simple mechanical cutter to mimic both the positions and diameters of the high abundance proteins spots. In the following it is called multi-aperture barrier.

In this latter implementation, the 2D gel is placed horizontally on an appropriate smooth substrate, e.g. metal plate or metal coated glass or plastic. It is covered by the multi-aperture barrier so that barrier holes are alligned with the high abundance protein spots. On the top of the multi-aperture barrier, an electrophoretic gel is placed which in the following is called covering gel. A transverse electrophoresis is initiated leading to removal of the proteins from "preparative 2D gel" to the covering gel in a direction perpendicular to both dimensions of previous electrophoresis in the 20 gel. Subsequently, the multi-aperture barrier and covering gel are removed. The preparative 20 gel is processed to liberate all remaining low abundance proteins, and the covering gel is used to liberate all high abundance proteins.

The mobility of proteins under transverse electrophoresis depends on their size. Thus in principle, The high abundance proteins of low mass will tend to be removed more efficiently than high abundance proteins of high mass. Thus, a method is disclosed in which in the step of multi-aperture barrier production, the aperture diameter is calculated taking in account both the spot diameter and expected protein mobility. For spots of the same diameter, i.e. the same abundance, the diameter of aperture in multi-aperture barrier will be smaller for low mass proteins than for high mass proteins.

In the described implementation, only the proteins and not the gel are removed from the bottom 2D-gel. Thus, instead of liberating all proteins from the preparative 2D gel, it can be reused. For example, one uses a longer than normal gel in the SDS-PAGE molecular weight direction. After electroelution of the high abundance proteins, all remaining proteins are re-sized by running the same gel for an additional time period. As all abundant proteins have been removed, the spatial resolution will improve after The second run because the overloading, both global and local, are no longer limiting factors.

### Capillary electrophoresis based application

Another preferred embodiment of this disclosure, which will also be explained with reference to the following figures, discloses principles to separate proteins, partitioned by abundance, from a complex mix such as a whole cell lysis or a biochemical cell fraction, by using capillary electrophoresis (CE).
- Figure 1:: a first embodiment of a CE separating device
- Figure 2 (A, B):: a second embodiment of a CE separating device

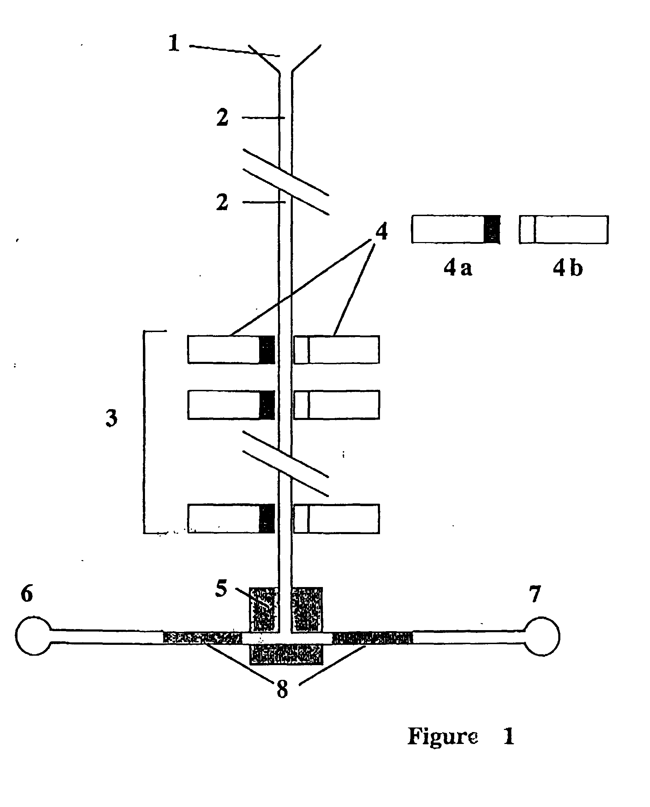

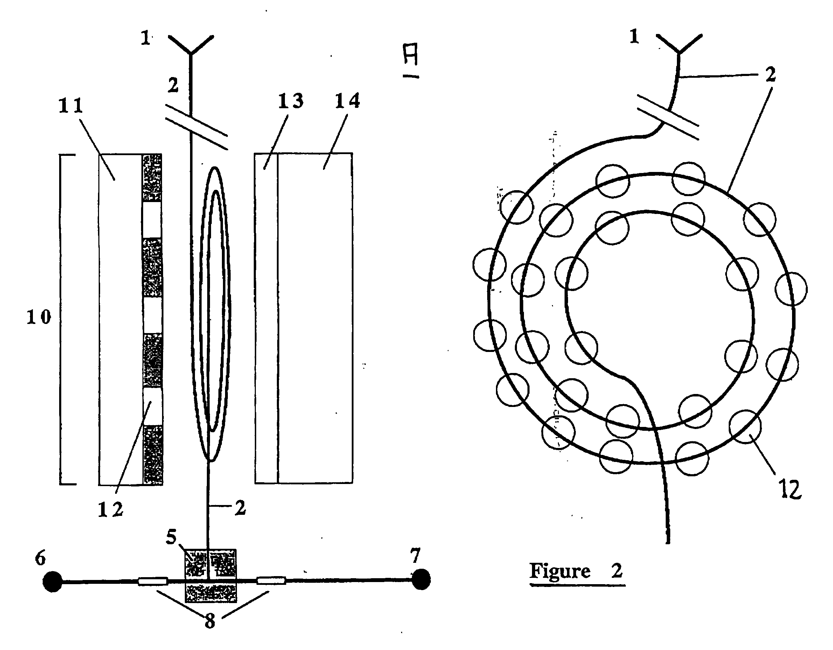

Figures 1 and 2 show two embodiments of the inventive CE separating device. The device according to Figure 1 comprises a fluid inlet 1, a capillary 2, a capillary region 3, where (e.g. a number of 16) detectors 4 each consisting of parts 4a and 4b are arranged, and a microchip 5 with fluidic channels where the fluid flow is gated into one of two channels (not designated by reference numbers). 4a and 4b could be a MPD detector which uses the same type, or different crystal types to detect photons. There could be multiple channels in one gate, or a series of chips one after the other to achieve higher gating capacity. For fluid to leave the system there are outlets 6 and 7 coupled to said channels. Electro-osmotic pumps or microvalves 8 are responsible for gating of fluid flow to either 6 or 7.

An appropriate device consists of a capillary, which could be kept at a certain temperature in a controlled manner, in particular the capillary could be cooled. Cooling is e.g. necessary to prevent carbamylation of primary amines in the urea buffer. Alternatively, if primary amines have been suitably derivatized, it is conceivable that increased heat may permit better analyte solubility. Ammonium cyanate produced under these conditions may be subsequently electrophoretically removed. Urea/CHAPS is suggested as the buffer since it is used in the isoelectric focusing step which follows. However other buffers could be employed. Proteins would be loaded in a buffer of, e.g. 9M urea, 4% CHAPS, of a pH to be optimized during development. In this buffer the proteins should be essentially denatured as described above, and not involved in any higher order molecular structures. Thus they migrate in CE as unique peaks. Use of suitable capillary wall derivatives minimizes the interaction of proteins with the CE wall. This is highly desirable to maintain the presence in solution of the low abundance analytes of interest. Preferably the detector 4 is a MPD Imager. After a suitable separation, e.g. by capillary zone electrophoresis, the analytes pass the detector 4 or its parts 4a and 4b, respectively. The flow rate and detection window should be optimized to allow maximum possible time in the detection window. Detection of attomol levels of analyte during an optimized CE run described below permits lower loading amounts than currently employed, permitting more highly resolved peaks and thus better resolution than presently used techniques. A gating mechanism (comprising 5 and 8) subsequent to the detector region 3 gates fractions toward alternative receptacles 6 and 7 based on the abundance of proteins. Said gating mechanism may operate by means of one or more microvalves, or by means of pumps causing fluidic flow into alternative channels, and thereby providing the gating function, which permits controlled deposition of e.g. nanoliter amounts of fluids into an array of receptacles. In a preferred embodiment of the invention the gating mechanism is isolated from the atmosphere to prevent evaporation of the small volumes involved. In a preferred version there would be several receptacles. In an especially preferred variant these are each coupled to an immobiline isoelectric focussing gradient.

In the case of CE it is expected that the flow velocity of a typical peak is about 30 seconds per mm. Assuming a peak size of few mm, and similar detector size, it is possible to establish reliably that the peak content is larger than about 20 attomol. At the level of about 0.1 femtomol a reasonable quantitation, say 10%, will be possible.

In a further preferred embodiment of this invention a linear array of MPD detectors is considered, each 5 mm in size, say a linear array of 16 detectors, as indicated in Fig. 1. It is known that peak formation during CE is achieved at a relatively long distance from the capillary exit. For example for a typical 1 m capillary the peaks are well formed at about 20 cm from the exit. Also, the velocity of peak propagation is known, and in any case can be calibrated to within a few percent. Thus with characteristic integration time of about 30 seconds, the same peak will be seen by subsequent detectors. Thus efficiently each peak will be seen 16 times by 16 different MPD detectors, and the total counting time will be about 10 minutes. Threshold detection can then be achieved for about one attomol per peak. This will also allow the reliable quantitation of peak height for peaks with about 10 attomol.

Both the geometry and dynamics of the peak propagation in the exit section of the capillary is thus known, and using appropriate algorithms the moment of a given peak exit from the capillary can be reliably predicted. This calculated time of exit, correlated with the information about peak height, is used to trigger an appropriate flow switch. Thus reliable assignment of all peaks larger than a given threshold (down to 1 attomol) is achieved. This concept of a concurrent measurement of multiple peaks in a multipixel MPD detector can be generalized to a 2D array of detectors. Such an MPD detector e.g. can quantitate the radiolabel readout of at least 49 separate crystals by a single spatially resolving photo multiplier (SR-PMT). In the here disclosed implementation the exit section of the capillary is wound in an appropriate spiral pattern, as embodied in Fig. 2. The detectors on a surface of the SR-PMT are placed with the same geometrical pattern as the above-said geometrical spiral. A full exit section of the capillary, say 20 cm, is concurrently measured by the MPD detectors. In Figure 2 the capillary 2 (connected to a fluid inlet 1) in form of a spiral has in a capillary (detection) region 10 an array of crystals 12 for MPD coupled to a single SR-PMT 11 and on the other (opposite) side one single large crystal 13 coupled to a non spatially resolved photomultiplier 14. All other parts of the device are designated as shown in Figure 1. Fig. 2A shows a side view of the device, Fig. 2B shows the arrangement of crystals 12 relative to capillary 2 in a top view. The arrangement of parts 11, 12, 13 and 14 forms a spatially resolving MPD (SR-MPD). This is a further improvement of detector sensitivity, and sub attomol peaks can be detected. Obviously a spiral is just one of the examples permitting mapping of a linear capillary on a two dimensional surface. Another possible geometry is a zig zag line meandering across the surface of SR-MPD.

In another preferred embodiment of the present invention a plurality of capillaries is coupled to a spatially resolved MPD with the detector pattern featuring n x m detectors. In such a configuration n capillaries are measured concurrently, each with m detectors. For example with a 13 x 13 array of detectors one can use 13 capillaries and detect about 10 attomole peaks. Such a configuration solves the throughput issue.

A preferred embodiment of the invention is an array of electrophoresis capillaries, which is coupled to a SR-MPD detector, consisting of a plurality of scintillators placed and read-out by a single SR-PMT, wherein the said scintillators are placed in an essentially quadratic grid pattern, i.e. each capillary is seen by the same row of detectors and the information from each row of detectors is used to activate a miniaturized fluidic valve.

### Combined 2D gel and capillary electrophoresis procedures

To overcome the possibility that low abundant proteins are masked by high abundant proteins and are removed together with the high abundant proteins, according to the invention the high abundance proteins are removed partially, say about to 90%. Thus, some amount of other proteins will be still present, though at very low abundance level. This is why the procedures for removal of high abundance proteins preferably comprise the use of highest possible sensitivity read-out methods, e.g. the use of sub-attomole sensitivity of MPD instrumentation.

There exist, however, other possibilities. In the case of mechanical removal or electroelution, the proteinic material from each spot is available and proteins are essentially not destroyed. Thus, the proteinic material from each of the high abundance protein spots is subsequently analyzed by any of a plurality of separation techniques such as CE, HPLC (high performance liquid chromatography), TLC (thin layer chromatography) or size exclusion filters. These methods are dependent on other physical or chemical characteristics of proteins than 2D gels. Thus, for each spot an efficient 3D separation can be obtained by performing subsequent 2D electrophoresis, high abundance of proteins spots removal, and a subsequent analytical separation step with CE, HPLC, or TLC. The most preferred combination is 2D gel followed by CE separation or vice versa.

By procedures described above, the high abundance fractions are eliminated and low abundance proteins returned to a pool of proteins to be re-analyzed by repeat of 2D electrophoresis. Optionally, the low abundance fractions are believed to be sufficiently well analyzed by the preparative 2D gel and CE step. The data are placed in an appropriate computer database, and fractions are forwarded for further steps of identification, e.g. by mass spectrometry or Edman degradation process.

It is possible, that within the single spot, there are only a few proteins. Thus, it will be possible to use separation methods with lower resolution than CE. Both HPLC and TLC have enough separation power. Herein, TLC is especially favorable as it is cheap, fast and a multilane method. Thus, throughput is adequate to perform post-analysis of hundreds of samples removed, spot by spot, from preparative 2D gel. Herein, the use of TLC/MPD with sub-attomole sensitivity is disclosed.

## Claims

1. Method for separation of components from a material to be analyzed, in particular from a biological material, characterized by at least one partition step partitioning said material into at least two components or at least two sets of components according to their relative abundances, said partition step being performed before further separation of components is done.

2. Method according to claim 1, characterized in that the high abundant components are removed in the partition step.

3. Method according to claim 1 or 2, characterized in that the components are biological molecules, in particular peptides, proteins, nucleic acids, lipids, and/or derivatives thereof, wherein the molecular weight of the biological molecules is especially between 1 and 1000 kDa.

4. Method according to one of the preceding claims, characterized in that the material to be analyzed comprises peptides and/or proteins of a proteom.

5. Method according to one of the preceding claims, characterized in that the total mass of the material to be analyzed is less than 1 mg, especially less than 1 µg.

6. Method according to one of the preceding claims, characterized in that said partition step is performed with the aid of affinity reagents, wherein the affinity reagents are especially collections of affinity reagents specific to the dominant components of the material to be analyzed.

7. Method according to claim 6, wherein said affinity reagents are antibodies, especially monoclonal antibodies, or aptamers.

8. Method according to one of claims 6 or 7, characterized in that said affinity reagents are arranged onto chips, wherein collections of affinity reagents are especially organized into arrays of spatially distinguishable pixels.

9. Method according to one of the preceding claims, characterized in that said partition step is performed by means of gel electrophoresis, especially 2D electrophoresis.

10. Method according to claim 9, characterized in that after said gel electrophoresis is performed at least one component following localization of said component is separated by removal, especially by excision, electroelution or laser induced evaporation.

11. Method according to claim 10, wherein localization and/or removal is performed computer aided and/or automated, especially by using a computer steered robot.

12. Method according to claim 10 or 11, characterized in that an electroelution tool, consisting of an appropriate semi-permeable electroelution probe, is brought into contact with the electrophoresis gel, and after placing under electric voltage, electroelution of at least one component is initiated.

13. Method according to one of claims 10 to 12, characterized in that a mask, especially computer generated, matching the pattern and diameters of spots, is placed on the gel supporting the removal of components, especially supporting an electroelution of components in a transverse electrophoresis.

14. Method according to one of the preceding claims, characterized in that said partition step is performed by means of capillary electrophoresis (CE).

15. Method according to claim 14, characterized in that components or sets of components are collected in different receptacles according to their abundances especially by using a gating mechanism.

16. Method according to claim 15, characterized in that signals of appropriate detectors are used to activate miniaturized fluidic switches according to a especially pre-set threshold of abundance.

17. Method according to one of claims 14 to 16, wherein at least one electrophoresis capillary, at least parts thereof, are wound into a pattern, especially a zig-zag or a spiral pattern.

18. Method according to one of the preceding claims, characterized in that components or sets of components obtained in a partition step are identified, especially by mass spectrometry, and a collection of affinity reagents used according to one of claims 6 to 8 is prepared or provided according to results of said identification.

19. Method according to one of the preceding claims, wherein, subsequent to said partition step according to the relative abundances, components are separated by means of gel electrophoresis, especially two dimensional (2D) gel electrophoresis, and/or are separated by means of capillary electrophoresis.

20. Method according to one of the preceding claims, wherein detection of the components in the partition step and/or in the subsequent separation is performed by means of multi photon detection (MPD) technology, especially using multipixel MPD detectors.

21. Method according to one of the preceding claims, wherein MPD detection is performed using multipixel detectors organised into a linear array of MPD detectors, especially an array of 16 MPD detectors, wherein said array is especially quantified by at least one spatially resolving photo multiplier.

22. Method according to one of the preceding claims, wherein MPD detection is performed using multipixel MPD detectors organised into a two dimensional array of MPD detectors, especially containing 49 or 100 separate scintillator cristals, wherein said array is especially quantified by at least one spatially resolving photo multiplier.

23. Method according to one of claims 20 to 22, wherein MPD detectors are placed in a grid pattern, especially a quadratic grid pattern.

24. Method according to one of claims 14 to 23, characterized in that an array of electrophoresis capillaries is coupled to at least one spatially resolving photo multiplier and every capillary is controlled by its own gating mechanism.

25. Apparatus for separation of components from material to be analyzed, comprising a device for gel electrophoresis and/or a device for capillary electrophoresis, characterized in that it is provided with a MPD device.

26. Apparatus according to claim 25, further characterized by a MPD device with at least one of the features of claims 20 to 23.

27. Apparatus according to the preamble of claim 25, especially according to one of claims 25 or 26, further characterized by a device for gel electrophoresis with one of the features of claims 9 to 13.

28. Apparatus according to the preamble of claim 25, especially according to one of claims 25 to 27, further characterized by a device for capillary electrophoresis with one of the features of claims 14 to 17.
